Europäisches Patentamt

European Patent Office

Office européen des brevets

(11) Numéro de publication : **0 630 579 A1**

# (12) DEMANDE DE BREVET EUROPEEN

(21) Numéro de dépôt : **94401406.7**

(51) Int. Cl.$^5$ : **A23K 1/16, C07C 51/41**

(22) Date de dépôt : **22.06.94**

(30) Priorité : **23.06.93 FR 9307627**

(43) Date de publication de la demande :
**28.12.94 Bulletin 94/52**

(84) Etats contractants désignés :
**AT BE CH DE DK ES GB GR IE IT LI LU NL PT SE**

(71) Demandeur : **COMPAGNIE CHIMIQUE D'AQUITAINE, Société Anonyme**
**Clos des Templiers,**
**Marchesseau,**
**Lalande de Pomerol**
**F-33500 Libourne (FR)**

(72) Inventeur : **Gossart, Philippe**
**Clos des Templiers**
**Marchesseau**
**33500 Lalande de Pomerol (FR)**

(74) Mandataire : **Derambure, Christian**
**Cabinet Bouju Derambure (Bugnion) S.A.,**
**52, rue de Monceau**
**F-75008 Paris (FR)**

(54) **Formulation de complement alimentaire pour animaux.**

(57) »L'invention concerne une formulation de complément alimentaire pour animaux sous forme solide. Elle comprend un mélange binaire de butyrate de sodium et de butyrate d'éthyle, la quantité de butyrate d'éthyle dans le mélange se situant entre 1 et 25 % en poids/poids en mélange total.

EP 0 630 579 A1

L'invention concerne une formulation de complément alimentaire pour animaux ainsi qu'une composition alimentaire pour animaux comprenant une telle formulation.

On sait déjà que des animaux consommant un régime maintenu ou complété en acide organique, d'une part, présentent un gain de poids plus élevé et, d'autre part, consomment plus d'aliments.

Ainsi, un certain nombre de travaux ont mis en évidence les effets positifs de l'acide citrique, de l'acide fumarique et de l'acide propionique sur l'engraissement des porcs (KIRCHGESSNER AND ROTH, fumaric acid as a feed additive in pig nutrition, Pig News and Information 3, 259-264, 1982 ; GIESTING AND EASTER, response of starter pigs to supplementation of corn soybean meal diets with organic acids. J. Anim. Sci. 60, 1288-1294, 1985).

L'état de la technique est illustré également par le document EP-B-0 233 819 qui décrit une formulation de complément alimentaire pour animaux comprennant (a) un mélange de sels de calcium et (1) d'acide isobutyrique et (2) d'au moins un acide choisi parmi les acides isovalériques, valériques et 2-méthyle butyrique, (b) de 5 à 15 % d'un additif comprenant de 60 à 100 % en poids d'huile de coprah ayant un indice d'iode supérieur à 5 et de 0 à 40 % en poids de suif.

Par ailleurs, différents travaux expérimentaux en alimentation animale montrent l'intérêt d'un complément alimentaire à base d'acide butyrique ou de ses sels sur de nombreux espèces animales.

Toutefois, ces travaux concluent généralement à une difficulté majeure : celle de la dissociation rapide dans le tractus digestif de l'acide butyrique en d'autres constituants à chaîne carbonnée plus courte.

Cette dissociation fait perdre tout l'intérêt de l'acide butyrique et naturellement ne permet pas à ce dernier d'atteindre les parties éloignées du tractus digestif, qui souvent répondent le mieux à l'action de l'acide butyrique.

Le problème à la base de l'invention est donc de proposer une formulation de complément alimentaire pour animaux dans laquelle l'acide butyrique est stabilisé sous sa forme $C_4$ et assurant aux animaux consommateurs d'un tel complément alimentaire de voir leur poids moyen s'accroître sensiblement.

A cet effet, l'invention propose une formulation de complément alimentaire pour animaux sous forme solide caractérisée en ce qu'elle comprend un mélange binaire de butyrate de sodium et de butyrate d'éthyle, la quantité de butyrate d'éthyle dans les mélanges se situant entre 1 et 25 % en poids par rapport au poids du mélange total.

D'autres caractéristiques et avantages de l'invention apparaîtront à la lecture de la description détaillée et des exemples qui suivront.

La formulation de complément alimentaire pour animaux, selon l'invention, est sous forme solide et comprend un mélange binaire de butyrate de sodium et de butyrate d'éthyle.

Une telle formulation permet notamment de stabiliser l'acide butyrique en sa forme $C_4$.

En effet, l'influence du butyrate de sodium sur le métabolisme animal est multiple.

Avant son absorption, il règle la composition de la flore épimurale (effet direct I) par l'inhibition de la prolifération de différentes bactéries (Eschérichia coli) ou en ayant aucun effet sur la division d'autres bactéries (Lactobacillus). Cela signifie que la composition de la flore épimurale est formée de façon plus optimale en présence de butyrate.

En outre, le butyrate a également un effet optimal au cours de son absorption. Il augmente l'échange $Na^+/H^+$ (effet direct II).

Le butyrate pénètre alors dans les cellules recouvrant le tractus digestif où il peut être transformé par métabolisme ou servir comme activateur de différents enzymes (effet direct III).

A la suite de son absorption, il est mis en contact, par la circulation sanguine, avec différentes cellules de l'organisme stimulant ainsi la secrétion de certaines hormones (insuline, glycagon ; effet direct IV).

Cette augmentation de la concentration en hormone peut engendrer une stimulation de la prolifération dans les cellules du tractus digestif (effet indirect V). Cette action indirecte du butyrate provoque une augmentation de la surface d'absorption du tractus digestif stimulant les mécanismes de transport.

Puisque la présence de butyrate augmente également sa propre production (effet I), l'administration orale de butyrate, même en petite quantité, a un rôle important. Le butyrate consommé augmente en quantité dans le tractus digestif au cours d'une réaction en chaîne. De plus, le butyrate administré oralement a également un effet optimal dans des parties éloignées du tractus digestif, qui souvent répondent le mieux à son action (par exemple, l'estomac).

Pour arriver à une telle action optimale, la quantité de butyrate d'éthyle dans le mélange binaire du type de l'invention, se situe entre 1 et 25 % en poids par rapport au poids du mélange total.

Notamment, la quantité de butyrate d'éthyle dans le mélange se situe entre 10 et 20 %, et plus particulièrement de l'ordre de 20 % en poids par rapport au poids du mélange total.

Selon une autre caractéristique de l'invention, le butyrate de sodium est synthétisé par neutralisation de l'acide butyrique par des sels de sodium de l'acide phosphorique choisi dans le groupe comprenant notamment le dihydrogénophosphate de sodium, le phosphate disodique, le phosphate trisodique.

Au cours de cette neutralisation de l'acide butyrique, le sel de sodium de l'acide phosphorique est choisi de façon à ce que le pKa de l'acide butyrique soit inférieur à la moyenne mathématique des deuxième et troisième constantes de dissociation de l'acide phosphorique.

Dans une variante préférée de l'invention, le butyrate de sodium est synthétisé par neutralisation de l'acide butyrique par du phosphate trisodique.

La synthèse du butyrate de sodium est effectuée à partir d'un mélange d'acide butyrique et de sels de l'acide phosphorique selon une proportion molaire de 2:1 respectivement.

Dans une variante préférée de l'invention, le butyrate de sodium est synthétisé à partir d'un mélange d'acide butyrique et de sels de l'acide phosphorique selon une proportion équimolaire.

Comme indiqué ci-dessus, une administration orale de la formulation de complément alimentaire selon l'invention est préférée, notamment sous forme de comprimés et/ou de granulés.

La formulation selon l'invention est appliquée à l'aliment en une quantité correspondant à une valeur comprise entre 0,1 et 10 % en poids de l'aliment.

Plus particulièrement, elle est appliquée à l'aliment en une quantité correspondant à de l'ordre de 0,17 % en poids de l'aliment.

Ainsi, si l'on compare cette concentration en butyrate de sodium à celles décrites dans la littérature relative à l'utilisation d'acide citrique, d'acide fumarique et d'acide propionique dans l'alimentation notamment porcine (de telles concentrations variant de 0,6 à 4,0 %), on remarque que le butyrate de sodium peut exercer son effet positif dans des concentrations de 3,6 à 24,2 fois plus faible que les acides organiques utilisés couramment.

L'invention propose également une composition alimentaire pour animaux comprenant la formulation complémentaire pour animaux dont les caractéristiques sont mentionnées ci-dessus.

Une telle composition alimentaire est appliquée à l'alimentation du bétail et/ou des porcs et/ou des volailles.

A titre d'exemple non limitatif, on peut mentionner les résultats expérimentaux suivants relatifs à la synthèse de butyrate de sodium à partir d'acide butyrique et de sels de sodium de l'acide phosphorique.

Les solutions suivantes ont été utilisées :
I. $Na_2HPO_4$ 2M et acide butyrique 1M
II. $Na_2HPO_4$ 1M et acide butyrique 1M
III. $Na_3PO_4$ 1M et acide butyrique 1M
IV. $Na_3PO_4$ 1M et acide butyrique 2M

Le but de ces expérimentations est d'examiner la neutralisation de l'acide butyrique (pour former le butyrate de sodium) avec $Na_2HPO_4$ ou $Na_3PO_4$, et d'étudier les conditions quantitatives de cette réaction.

Dans ce but, les conditions opératoires sont les suivantes :

- quantité de sels de sodium de l'acide phosphorique utilisée : 5000 mg.
- détermination du pH de l'échantillon : 360 mg des échantillons séchées à l'air obtenus sont dissous dans de l'eau distillée, on mesure le pH de la solution résultante et on la titre avec 0,1 ml d'HCl 1N (pH 2).
- détermination de la concentration en acide butyrique : 500 mg des échantillons sont dissous dans 200 ml d'eau distillée (2,5 mg/cm³). A la solution ainsi obtenue, on ajoute dans une quantité de 20 %, 1 ml d'acide oxalique 0,6 M et 5 ml de l'échantillon. Après centrifugation, la concentration d'acide butyrique libérée est déterminée par chromatograhie en phase gazeuse.

Des quantités connues d'acide butyrique et de sels de sodium de l'acide phosphorique sont mélangés sans refroidissement et maintenues à température ambiante pendant 6 jours. Des analyses sont effectuées le premier, troisième et sixième jour.

## EXEMPLE I

5000 mg de $Na_2HPO_4.2H_2O$ (28,1 mM)
+ 1293 µl d'acide butyrique (14,1 mM)
   . rapport molaire : $Na_2HPO_4.2H_2O$/acide butyrique = 2/1
   . pH d'une solution aqueuse à 0,5 % le 1er jour : 8,92
   . teneur en acide butyrique le 1er jour : 0,01 mM/l
Résultat : pas de réaction.

## EXEMPLE II

5000 mg de $Na_2HPO_4.2H_2O$ (28,1 mM)
+ 2586 µl d'acide butyrique (28,1 mM)
   . rapport molaire : $Na_2HPO_4.2H_2O$/acide butyrique = 1/1
   . pH d'une solution aqueuse à 0,5 % le 1er jour : 7,11
   . pH d'une solution aqueuse à 0,5 % le 3ème jour : 8,82
   . teneur en acide butyrique le 1er jour : 7,69 mM/l
   . teneur en acide butyrique le 3ème jour : 0,47 mM/l
   . teneur en acide butyrique le 6ème jour : 0,23 mM/l
Résultat : pas de réaction.

## EXEMPLE III

5000 mg de $Na_3PO_4.12H_2O$ (13,2 mM)
+ 1214 µl d'acide butyrique (13,2 mM)
   . rapport molaire : $Na_3PO_4.12H_2O$/acide butyrique = 1/1
   . pH d'une solution aqueuse à 0,5 % le 3ème jour : 10,58
   . teneur en acide butyrique le 3ème jour : 10,76 mM/l
   . teneur en acide butyrique le 1er jour : 9,12 mM/l

Résultat : l'acide butyrique est transformé à 100 % en butyrate de sodium.

## EXEMPLE IV

5000 mg de $Na_3PO_4.12H_2O$ (13,2 mM)
+ 2429 µl d'acide butyrique (13,2 mM)
.  rapport molaire : $Na_3PO_4.12H_2O$/acide butyrique = 1/2
.  pH d'une solution aqueuse à 0,5 % le 3ème jour : 7,97
.  teneur en acide butyrique le 3ème jour : 11,95 mM/l
.  teneur en acide butyrique le 1er jour : 10,15 mM/l

Résultat : l'acide butyrique est transformé à 50 % en butyrate de sodium.

Les conclusions que l'on peut tirer de ces exemples sont les suivantes :

1°) l'acide butyrique étant plus faible que l'acide phosphorique, $Na_3PO_4$ ne peut être totalement libéré de son sel par l'acide butyrique.

Sur la base des résultats des exemples I à IV exposés ci-dessus, on remarque que la réaction $Na_3PO_4 \rightarrow Na_2HPO_4$ a lieu (exemples III et IV) ; alors que la réaction $Na_2HPO_4 \rightarrow NaH_2PO_4$ n'a pas lieu (exemples I et II).

Ce résultat peut s'expliquer de la manière suivante : si l'on veut libérer $Na_3PO_4$ de son sel, la constante de dissociation de l'acide butyrique (pKa = 4,82) doit être plus faible que celle de l'acide phosphorique ou de ses points équivalents.

Comme l'acide phosphorique a trois constances de dissociation ($pKa_1$ = 2,12 ; $pKa_2$ = 7,21 ; $pKa_3$ = 11,75), leur moyenne arithmatique donnent ses équivalents (premier point équivalent : moyenne arithmétique de $pKa_1$ et $pKa_2$ = 4,67 ; deuxième point équivalent : moyenne arithmétique de $pKa_2$ et $pKa_3$ = 9,48), lesquelles déterminent les réactions chimiques ayant lieu.

Or, on remarque que le pKa de l'acide butyrique est supérieur au premier point équivalent de l'acide phosphorique ; en d'autres termes :
pKa (acide butyrique) > 1/2 ($pKa_1$ + $pKa_2$)

Par contre, le pKa de l'acide butyrique est inférieur au second point équivalent de l'acide phosphorique ; en d'autres termes :
pKa (acide butyrique) < 1/2 ($pKa_2$ + $pKa_3$)

Pour cette raison, seule la première réaction mentionnée ci-dessus peut prendre place.

2°) Bien qu'une mole de $Na_3PO_4$ est capable de neutraliser une môle d'acide butyrique, les quantités de composés décrits dans l'exemple III sont les plus adequates pour la neutralisation de l'acide butyrique et, de ce fait, pour la mise en oeuvre de la formulation selon l'invention.

3°) Si l'acide butyrique- ne forme pas de butyrate de sodium, il n'est pas stable et est volatile à température ambiante.

## Revendications

1.  Formulation de complément alimentaire pour animaux sous forme solide, caractérisée en ce qu'elle comprend un mélange binaire de butyrate de sodium et de butyrate d'éthyle, la quantité de butyrate d'éthyle dans le mélange se situant entre 1 et 25 % en poids/poids en mélange total.

2.  Formulation de complément alimentaire selon la revendication 1, caractérisée en ce que la quantité de butyrate d'éthyle dans le mélange se situe entre 10 et 20 % et plus particulièrement de l'ordre de 20 % en poids/poids de mélange total.

3.  Formulation de complément alimentaire selon la revendication 1 ou 2, caractérisée en ce que le butyrate de sodium est synthétisé par neutralisation de l'acide butyrique par des sels de sodium de l'acide phosphorique choisis dans le groupe comprenant notamment le dihydrogénophosphate de sodium, le phosphate disodique, le phosphate trisodique.

4.  Formulation de complément alimentaire selon la revendication 3, caractérisée en ce que le sel de sodium de l'acide phosphorique est choisi de façon à ce que le pKa de l'acide butyrique soit inférieur à la moyenne arithmétique des deuxième et troisième constantes de dissociation de l'acide phosphorique.

5.  Formulation de complément alimentaire selon la revendication 3 ou 4, caractérisée en ce que le butyrate de sodium est synthétisé par neutralisation de l'acide butyrique par du phosphate trisodique.

6.  Formulation de complément alimentaire selon l'une quelconque des revendications 3 à 5, caractérisée en ce que le butyrate de sodium est synthétisé à partir d'un mélange d'acide butyrique et de sels de l'acide phosphorique selon une proportion equimolaire.

7.  Formulation de complément alimentaire selon l'une quelconque des revendications 3 à 5, caractérisée en ce que le butyrate de sodium est synthétisé à partir d'un mélange d'acide butyrique et de sels de l'acide phosphorique selon une proportion molaire de 2 : 1 respectivement.

8.  Formulation de complément alimentaire selon l'une quelconque des revendications 1 à 7, caractérisée en ce qu'elle se présente sous forme de comprimés et/ou de granulés.

9. Formulation de complément alimentaire selon l'une quelconque des revendications 1 à 8, caractérisée en ce qu'elle est appliquée à l'aliment en une quantité correspondant à une valeur comprise entre 0,1 et 10 % en poids de l'aliment.

10. Formulation de complément alimentaire selon la revendication 9, caractérisée en ce qu'elle est appliquée à l'aliment en une quantité correspondant à de l'ordre de 0,17 % en poids de l'aliment.

11. Composition alimentaire pour animaux comprenant la formulation de complément alimentaire pour animaux selon l'une quelconque des revendications 1 à 10.

12. Composition alimentaire pour animaux selon la revendication 11, caractérisée en ce qu'elle est appliquée à l'alimentation du bétail et/ou des porcs et/ou des volailles.

| | Office européen des brevets | RAPPORT DE RECHERCHE EUROPEENNE | Numero de la demande EP 94 40 1406 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 424 055 (UNILEVER PLC) <br> * le document en entier * <br> --- | 1,8-12 | A23K1/16 <br> C07C51/41 |
| A | ACTA VETERINARIA HUNGARICA, <br> vol.38, no.1-2, 1990, HU <br> pages 3 - 17 <br> P. GALFI ET AL. 'Feeding trial in pigs <br> with a diet containing sodium n-butyrate' <br> * le document en entier * <br> --- | 1,8-12 | |
| A | FR-A-2 141 423 (CELANESE CORPORATION) <br> * revendications 1,6,13,15-17,20,22 * <br> --- | 1,8-12 | |
| A | WO-A-84 00668 (EASTMAN KODAK COMPANY) <br> * page 4, ligne 30 - ligne 34 * <br> * revendications 1-3 * <br> --- | 1 | |
| A | GB-A-1 141 464 (FEED SERVICE (LIVESTOCK)) <br> * page 1, ligne 48 - ligne 53 * <br> --- | 1 | |
| A | DATABASE WPI <br> Week 7544, <br> Derwent Publications Ltd., London, GB; <br> AN 75-73240W <br> & JP-A-50 069 244 (HASEGAWA T CO KK) 10 <br> Juin 1975 <br> * abrégé * <br> ----- | 1 | DOMAINES TECHNIQUES RECHERCHES (Int.Cl.5) <br><br> A23K <br> C07C |

Le présent rapport a été établi pour toutes les revendications

| Lieu de la recherche | Date d'achèvement de la recherche | Examinateur |
|---|---|---|
| LA HAYE | 16 Août 1994 | Dekeirel, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)